# EUROPEAN PATENT SPECIFICATION

(11) **EP 2 640 297 B1**
(45) Date of publication and mention of the grant of the patent: **30.03.2016**
(21) Application number: 11788322.3
(22) Date of filing: 18.11.2011
(51) Int. Cl.: A61N 1/36, A61B 18/00, A61B 18/12, A61N 7/02, A61B 18/14

(54) **RENAL NERVE DETECTION AND ABLATION APPARATUS**
VORRICHTUNG FÜR NIERENNERVENDETEKTION UND ABLATION
APPAREIL DE DÉTECTION ET D'ABLATION DE NERF RÉNAL

(30) Priority: 19.11.2010 US 415579 P
(43) Date of publication of application: 25.09.2013
(73) Proprietor: Boston Scientific Scimed, Inc., Maple Grove, MN 55311 (US)
(72) Inventor: CROW, Loren, M, Los Gatos, CA 95030 (US); JENSON, Mark, L., Greenfield, MN 55357 (US); SMITH, Scott, Chaska, MN 55318 (US)
(74) Representative: Vossius & Partner Patentanwälte Rechtsanwälte mbB
(86) International application number: PCT/US2011/061417
(87) International publication number: WO 2012/068471

(56) References cited:
- WO-A2-2006/041881
- US-A- 4 830 003
- US-A- 5 170 802
- US-A- 5 916 239
- US-A1- 2003 216 792
- US-A1- 2010 168 739

## Description

### SUMMARY

The present dislosure is directed to apparatuses and methods for detecting renal nerves and ablating detected renal nerves. The present invention is defined in claim1. Preferred embodiments are defined by dependent claims 2-12. According to various embodiments, an apparatus includes a catheter comprising a flexible shaft having a proximal end, a distal end, and a length sufficient to access a patient's renal artery relative to a percutaneous access location. An electrode array is provided at the distal end of the shaft and dimensioned for deployment within the renal artery. The electrode array is transformable between a low-profile introduction configuration and a deployed configuration. The electrode array includes a plurality of spaced-apart electrodes positionable at a plurality of renal artery sites at or near a wall of the renal artery when in the deployed configuration. An external system is configured to couple to the catheter and deliver stimulation energy selectively to the multiplicity of electrodes for eliciting a physiologic response from the patient but insufficient to ablate renal nerves. The external system is further configured to deliver high-frequency electrical energy to electrodes at target renal artery sites that elicit the physiologic response for ablating renal nerves proximate the target sites.

There is also disclosed a method involving delivering stimulation energy to one or more renal artery sites in accordance with a predetermined energy delivery protocol, the stimulation energy sufficient to elicit a physiologic response from the patient but insufficient to ablate renal nerves. The method also involves identifying target sites of the renal artery sites that elicit the physiologic response, and ablating renal nerve tissue at the target sites.

In accordance with some embodiments, an apparatus includes a stimulation catheter and an ablation catheter. The stimulation catheter includes a flexible shaft having a proximal end, a distal end, and a length sufficient to access a patient's renal artery relative to a percutaneous access location. A stimulation arrangement is provided at the distal end of the shaft and configured to deliver a stimulation agent to a multiplicity of renal artery sites. The ablation catheter includes a flexible shaft having a proximal end, a distal end, and a length sufficient to access the patient's renal artery relative to a percutaneous access location. The ablation catheter includes an ablation arrangement provided at the distal end of the ablation catheter's shaft. A first external system is configured to couple to the stimulation catheter and facilitate delivery of the stimulation agent selectively to a plurality of renal artery sites for eliciting a physiologic response from the patient. A second external system is configured to couple to the ablation catheter and facilitate delivery of an ablative agent to target renal artery sites that elicit the physiologic response for ablating renal nerves proximate the target sites.

The stimulation catheter and the ablation catheter can be separate devices, or a single device can have both stimulation and ablation arrangements. The stimulation and ablation arrangements can use similar technology (e.g., electrodes and use of electrical energy to accomplish their functions), or different technologies (e.g., one can be electrical RF and the other can be ultrasound). The stimulation and ablation arrangements can utilize common elements (e.g., the same electrode can be used for applying stimulation energy as for applying RF ablation energy) or the same transducer can apply a low-energy acoustic stimulation energy and a high-energy acoustic ablation energy, for example.

Catheters for renal neuromodulation are disclosed in WO2006/041881 A2. These catheters comprise helically expanding electrodes or an electrode carrying basket which may be self-expanding or activity expanded.

These and other features can be understood in view of the following detailed discussion and the accompanying drawings.

### BRIEF DESCRIPTION OF THE DRAWINGS

Figure 1 is an illustration of a right kidney and renal vasculature including a renal artery branching laterally from the abdominal aorta;
Figures 2A and 2B illustrate sympathetic innervation of the renal artery;
Figure 3A illustrates various tissue layers of the wall of the renal artery;
Figures 3B and 3C illustrate a portion of a renal nerve;
Figure 4 is a flow chart illustrating various processes of a renal nerve detection and ablation methodology in accordance with various embodiments;
Figure 5 shows an apparatus for detecting and ablating renal nerves in accordance with various embodiments;
Figures 6-9 illustrate ablation catheters that include one or more self-expanding basket structures that support a multiplicity of electrodes configured for detecting and ablating renal nerves in accordance with various embodiments; Figures 6 and 7 showing embodiments of the present invention and
Figures 10 and 11 illustrate ablation catheters that include a multiplicity of resilient apposition wires that support a multiplicity of electrodes configured for detecting and ablating renal nerves in accordance with various embodiments.

### DETAILED DESCRIPTION

Ablation of perivascular renal nerves has been used for treatment of hypertension. There is presently no effective way of reliably locating the target renal nerves, so conventional approaches sought to ablate at arbitrary locations spaced axially and circumferentially apart to reduce the risk of arterial stenosis. This approach can ablate more tissue than is required, but can also miss significant target nerves, because the nerves can follow an unpredictable and meandering path generally along the arterial adventitia. Significant renal nerves and nerve bundles can easily be missed using conventional approaches. As such, conventional approaches are less effective than desired, and cause more unnecessary injury to non-target tissues than desired. There is also no good way of determining whether the target nerves have been successfully ablated, so the clinician does not know whether the procedure is complete.

Embodiments of the disclosure are directed to apparatuses and methods for stimulating perivascular renal nerves to locate renal nerves and determine target nerve ablation sites for more effective treatment of hypertension. Embodiments of the disclosure utilize pain or other signals (e.g., physiologic responses) resulting from stimulation of the target renal nerves to detect which tissue locations are best to target, and whether the target nerves have been ablated.

According to various embodiments, a catheter has a working portion with multiple independent electrodes which is advanced into position in the renal artery. The electrodes are deployed at or near the artery wall, spaced axially and/or circumferentially apart in discrete locations. Each electrode is energized one at a time or in predetermined combinations, using low stimulation energy that will not ablate tissue, to detect whether there is sufficient nerve response to indicate that target nerves are in the vicinity of that particular electrode.

The electrode is energized with frequency, amplitude, and pattern of electrical energy that enhances eliciting of a physiologic response. The response can be pain, tingling, heat, pressure, or other physiologic indicators of sympathetic nerve activity. After cycling through the multiple electrodes, RF ablation is performed at those sites confirmed to be near target nerves. The catheter can remain in position, performing RF ablation using the specific electrodes determined to be at the more effective locations, avoiding the need for repositioning and complicated location indexing.

After ablation, selected excitation and sensing can be repeated to immediately confirm the ablation effect on the target nerves. In addition to sensations like pain, tingling, warmth or pressure, the sensed response can be any changes in sympathetic signals or a resulting cascade of responses, including chemical or electrical changes, biometric indicators such as skin conductivity or sweating, blood pressure, pulse or respiratory changes, changes in vascular or muscle tone, autonomic gastrointestinal activity, pupil response, cardiac electrical activity, and the like.

Specific excitation of an autonomic nerve by an electrode can result in CNS-mediated autonomic nerve signals which can be measured in other locations, such as an afferent nerve signal causing excitation of an efferent signal, to increase smooth muscle tone, for example. Certain tissues affected by autonomic nerves can in turn provide information or response to somatic, sensory, or motor nerves, which can be detected, such as the pelvic nerves for example.

A response to the nerve stimulation can be sensed locally or remotely by electrodes and/or one or more sensors on the catheter or another device such as a guide catheter or sheath, or by skin sensors, EKG or respiration (via a transthoracic impedance or minute ventilation sensor) or EEG or EMG or blood pressure monitoring (external or vascular sensor), blood gas concentration, artery blood flow, blood chemistry, or visual changes, for example. Signals produced by one or more electrodes or sensors positioned in the aorta, for example, can be monitored by the catheter to detect vascular smooth muscle changes, or to detect overall splanchnic sympathetic activity. An expandable ring, collar, or basket structure can be provided on a guide catheter for this purpose. The response can be measured nerve activity, measured by a separate monitoring electrode, or by other electrodes or sensors on the catheter, or measured at other locations such as along the spinal cord or near a peripheral nerve.

In various configurations, a multiplicity of electrodes are arranged at different axial and circumferential positions in the renal artery. The number of electrodes can be relatively small or relatively large, for example. A control unit of an energy delivery system cycles through single or combinations of electrodes and analyzes the response to stimulation of the various electrodes, and determines the optimal choice of sites to provide the most effect in ablating the target nerves, with the least risk of artery heating in adjacent locations to reduce the chance for significant arterial injury, spasm or stenosis. The selected ablation sites are then treated with high-power RF energy to ablate the target nerves. One or more sensors can be incorporated in the electrode array to sense one or more parameters at the electrode-tissue interface. Representative sensors include temperature, impedance, voltage, acoustic, pressure, plethysmography, pulse oximetry, strain, blood chemistry, and tissue stiffness (elasticity) sensors, for example. Sensor signals may be used to automatically moderate and terminate the ablation procedure.

In another configuration, electrodes are arranged in a spiral pattern at different circumferential positions along a length of the renal artery. The catheter is repositioned to determine which locations provide optimal effect. According to some embodiments, an external sheath retains the apparatus in a low-profile configuration for introduction and advancement in the vasculature.

In other embodiments, many electrodes are simultaneously used, monitoring nerve response at many locations. An external control unit is used to interpret the response from each location, and determines which electrodes should be energized for ablation to maximize the effect on the target nerves while minimizing artery heating in adjacent locations to reduce the chance for any significant spasm or stenosis response.

Various embodiments include a self-expanding basket structure, provided as a distal end of the catheter, which supports a multiplicity of electrodes in an axially and circumferentially spaced-apart relationship. One end of the basket structure is fixedly mounted to the catheter's shaft, and the other end is affixed to a sliding attachment member which allows the free end of the basket structure to translate longitudinally along the shaft to facilitate radial expansion and reduction of the basket structure. In some embodiments, multiple self-expanding basket structures are provided at a catheter's distal end, with one end of the multiple basket structures fixedly mounted to the catheter's shaft, while the other is free to move axially along the shaft. A sliding attachment member is situated between each of the basket structures and at the free end of the multiple basket structures. In other embodiments, a shorter, simpler basket configuration can be used in multiple axial locations. This approach requires accurate device repositioning, but has the advantage of a simpler device and shorter cage structure. A sheath can be used to constrain the electrodes and basket(s) during delivery to the renal artery.

In accordance with some embodiments, two separate vascular devices can be used, with one device having the sensing electrodes, and the other having one or more ablation electrodes. The nerve stimulation can utilize electrical energy as previously described, or other stimulation mechanisms or energies, or combinations of stimulation mechanisms and energies can be utilized. For example, renal nerves can be stimulated using a variety of mechanisms and energies, non-limiting examples of which include the following: visible, UV, or IR light or laser light; magnetism; sonic or ultrasonic energy, low-frequency vibration or mechanical impact loading, heating or cooling, microwave or RF energy, neurotransmitter or other chemical or drug, by pressure changes, or osmotic or pH changes, or by specific patterns or changes in these or other stimulatory mechanisms.

A two-part sensing approach can be used, in which one type of signal is optimized to block pain, and another type of signal is used which would normally cause pain. For example, temporary heating, an infusion of neurogenically painful chemical, or a neurotransmitter can be delivered to the renal artery, followed by blocking and incrementally unblocking certain locations of the renal artery to reveal which locations are effective for ablation targets. In another embodiment, using painful stimulation from a distal electrode, and blocking the nerve signal from various proximal locations, may discriminate which locations should be ablated for afferent nerves. A proximal stimulation, which would normally cause temporary vasoconstriction by nerve activation of arterial smooth muscle cells, may be used to locate the most significant perivascular nerve bundles. Stimulation, followed by blocking at various distal locations, may be used to detect the location of efferent nerves. Stimulation and detection of nerve signals may be used, analogous to an electromyelogram. Pairs of electrodes may be used. Each pair can have a proximal and a distal electrode at similar circumferential positions, to detect where significant nerve transmission occurs, without requiring significant pain for detection.

Various embodiments of the disclosure are directed to apparatuses and methods for renal denervation for treating hypertension. Hypertension is a chronic medical condition in which the blood pressure is elevated. Persistent hypertension is a significant risk factor associated with a variety of adverse medical conditions, including heart attacks, heart failure, arterial aneurysms, and strokes. Persistent hypertension is a leading cause of chronic renal failure. Hyperactivity of the sympathetic nervous system serving the kidneys is associated with hypertension and its progression. Deactivation of nerves in the kidneys via renal denervation can reduce blood pressure, and may be a viable treatment option for many patients with hypertension who do not respond to conventional drugs.

The kidneys are instrumental in a number of body processes, including blood filtration, regulation of fluid balance, blood pressure control, electrolyte balance, and hormone production. One primary function of the kidneys is to remove toxins, mineral salts, and water from the blood to form urine. The kidneys receive about 20-25% of cardiac output through the renal arteries that branch left and right from the abdominal aorta, entering each kidney at the concave surface of the kidneys, the renal hilum.

Blood flows into the kidneys through the renal artery and the afferent arteriole, entering the filtration portion of the kidney, the renal corpuscle. The renal corpuscle is composed of the glomerulus, a thicket of capillaries, surrounded by a fluid-filled, cup-like sac called Bowman's capsule. Solutes in the blood are filtered through the very thin capillary walls of the glomerulus due to the pressure gradient that exists between the blood in the capillaries and the fluid in the Bowman's capsule. The pressure gradient is controlled by the contraction or dilation of the arterioles. After filtration occurs, the filtered blood moves through the efferent arteriole and the peritubular capillaries, converging in the interlobular veins, and finally exiting the kidney through the renal vein.

Particles and fluid filtered from the blood move from the Bowman's capsule through a number of tubules to a collecting duct. Urine is formed in the collecting duct and then exits through the ureter and bladder. The tubules are surrounded by the peritubular capillaries (containing the filtered blood). As the filtrate moves through the tubules and toward the collecting duct, nutrients, water, and electrolytes, such as sodium and chloride, are reabsorbed into the blood.

The kidneys are innervated by the renal plexus which emanates primarily from the aorticorenal ganglion. Renal ganglia are formed by the nerves of the renal plexus as the nerves follow along the course of the renal artery and into the kidney. The renal nerves are part of the autonomic nervous system which includes sympathetic and parasympathetic components. The sympathetic nervous system is known to be the system that provides the bodies "fight or flight" response, whereas the parasympathetic nervous system provides the "rest and digest" response. Stimulation of sympathetic nerve activity triggers the sympathetic response which causes the kidneys to increase production of hormones that increase vasoconstriction and fluid retention. This process is referred to as the renin-angiotensin-aldosterone-system (RAAS) response to increased renal sympathetic nerve activity.

In response to a reduction in blood volume, the kidneys secrete renin, which stimulates the production of angiotensin. Angiotensin causes blood vessels to constrict, resulting in increased blood pressure, and also stimulates the secretion of the hormone aldosterone from the adrenal cortex. Aldosterone causes the tubules of the kidneys to increase the reabsorption of sodium and water, which increases the volume of fluid in the body and blood pressure.

Congestive heart failure (CHF) is a condition that has been linked to kidney function. CHF occurs when the heart is unable to pump blood effectively throughout the body. When blood flow drops, renal function degrades because of insufficient perfusion of the blood within the renal corpuscles. The decreased blood flow to the kidneys triggers an increase in sympathetic nervous system activity (i.e., the RAAS becomes too active) that causes the kidneys to secrete hormones that increase fluid retention and vasorestriction. Fluid retention and vasorestriction in turn increases the peripheral resistance of the circulatory system, placing an even greater load on the heart, which diminishes blood flow further. If the deterioration in cardiac and renal functioning continues, eventually the body becomes overwhelmed, and an episode of heart failure decompensation occurs, often leading to hospitalization of the patient.

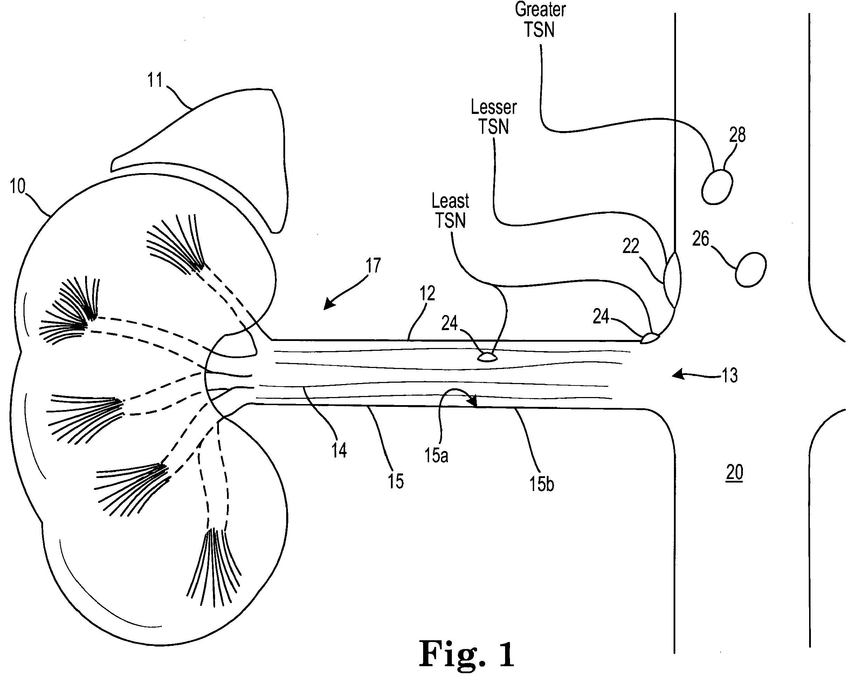

Figure 1 is an illustration of a right kidney 10 and renal vasculature including a renal artery 12 branching laterally from the abdominal aorta 20. In Figure 1, only the right kidney 10 is shown for purposes of simplicity of explanation, but reference will be made herein to both right and left kidneys and associated renal vasculature and nervous system structures, all of which are contemplated within the context of embodiments of the disclosure. The renal artery 12 is purposefully shown to be disproportionately larger than the right kidney 10 and abdominal aorta 20 in order to facilitate discussion of various features and embodiments of the present disclosure.

The right and left kidneys are supplied with blood from the right and left renal arteries that branch from respective right and left lateral surfaces of the abdominal aorta 20. Each of the right and left renal arteries is directed across the crus of the diaphragm, so as to form nearly a right angle with the abdominal aorta 20. The right and left renal arteries extend generally from the abdominal aorta 20 to respective renal sinuses proximate the hilum 17 of the kidneys, and branch into segmental arteries and then interlobular arteries within the kidney 10. The interlobular arteries radiate outward, penetrating the renal capsule and extending through the renal columns between the renal pyramids. Typically, the kidneys receive about 20% of total cardiac output which, for normal persons, represents about 1200 mL of blood flow through the kidneys per minute.

The primary function of the kidneys is to maintain water and electrolyte balance for the body by controlling the production and concentration of urine. In producing urine, the kidneys excrete wastes such as urea and ammonium. The kidneys also control reabsorption of glucose and amino acids, and are important in the production of hormones including vitamin D, renin and erythropoietin.

An important secondary function of the kidneys is to control metabolic homeostasis of the body. Controlling hemostatic functions include regulating electrolytes, acid-base balance, and blood pressure. For example, the kidneys are responsible for regulating blood volume and pressure by adjusting volume of water lost in the urine and releasing erythropoietin and renin, for example. The kidneys also regulate plasma ion concentrations (e.g., sodium, potassium, chloride ions, and calcium ion levels) by controlling the quantities lost in the urine and the synthesis of calcitrol. Other hemostatic functions controlled by the kidneys include stabilizing blood pH by controlling loss of hydrogen and bicarbonate ions in the urine, conserving valuable nutrients by preventing their excretion, and assisting the liver with detoxification.

Also shown in Figure 1 is the right suprarenal gland 11, commonly referred to as the right adrenal gland. The suprarenal gland 11 is a star-shaped endocrine gland that rests on top of the kidney 10. The primary function of the suprarenal glands (left and right) is to regulate the stress response of the body through the synthesis of corticosteroids and catecholamines, including cortisol and adrenaline (epinephrine), respectively. Encompassing the kidneys 10, suprarenal glands 11, renal vessels 12, and adjacent perirenal fat is the renal fascia, e.g., Gerota's fascia, (not shown), which is a fascial pouch derived from extraperitoneal connective tissue.

The autonomic nervous system of the body controls involuntary actions of the smooth muscles in blood vessels, the digestive system, heart, and glands. The autonomic nervous system is divided into the sympathetic nervous system and the parasympathetic nervous system. In general terms, the parasympathetic nervous system prepares the body for rest by lowering heart rate, lowering blood pressure, and stimulating digestion. The sympathetic nervous system effectuates the body's fight-or-flight response by increasing heart rate, increasing blood pressure, and increasing metabolism.

In the autonomic nervous system, fibers originating from the central nervous system and extending to the various ganglia are referred to as preganglionic fibers, while those extending from the ganglia to the effector organ are referred to as postganglionic fibers. Activation of the sympathetic nervous system is effected through the release of adrenaline (epinephrine) and to a lesser extent norepinephrine from the suprarenal glands 11. This release of adrenaline is triggered by the neurotransmitter acetylcholine released from preganglionic sympathetic nerves.

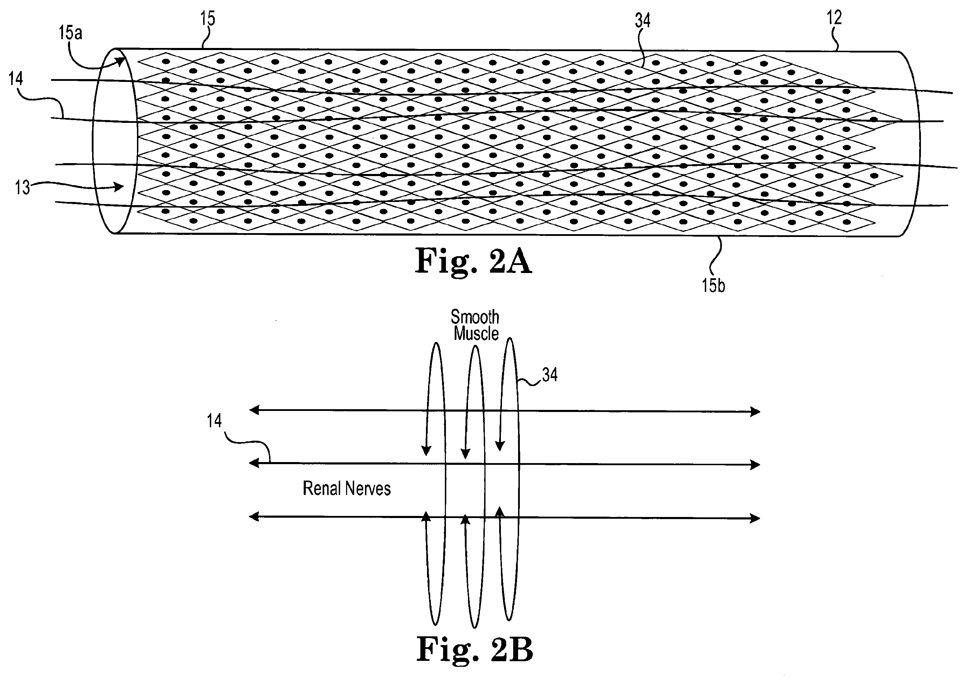

The kidneys and ureters (not shown) are innervated by the renal nerves 14. Figures 1 and 2A-2B illustrate sympathetic innervation of the renal vasculature, primarily innervation of the renal artery 12. The primary functions of sympathetic innervation of the renal vasculature include regulation of renal blood flow and pressure, stimulation of renin release, and direct stimulation of water and sodium ion reabsorption.

Most of the nerves innervating the renal vasculature are sympathetic postganglionic fibers arising from the superior mesenteric ganglion 26. The renal nerves 14 extend generally axially along the renal arteries 12, enter the kidneys 10 at the hilum 17, follow the branches of the renal arteries 12 within the kidney 10, and extend to individual nephrons. Other renal ganglia, such as the renal ganglia 24, superior mesenteric ganglion 26, the left and right aorticorenal ganglia 22, and celiac ganglia 28 also innervate the renal vasculature. The celiac ganglion 28 is joined by the greater thoracic splanchnic nerve (greater TSN). The aorticorenal ganglia 26 is joined by the lesser thoracic splanchnic nerve (lesser TSN) and innervates the greater part of the renal plexus.

Sympathetic signals to the kidney 10 are communicated via innervated renal vasculature that originates primarily at spinal segments T10-T12 and L1. Parasympathetic signals originate primarily at spinal segments S2-S4 and from the medulla oblongata of the lower brain. Sympathetic nerve traffic travels through the sympathetic trunk ganglia, where some may synapse, while others synapse at the aorticorenal ganglion 22 (via the lesser thoracic splanchnic nerve, i.e., lesser TSN) and the renal ganglion 24 (via the least thoracic splanchnic nerve, i.e., least TSN). The postsynaptic sympathetic signals then travel along nerves 14 of the renal artery 12 to the kidney 10. Presynaptic parasympathetic signals travel to sites near the kidney 10 before they synapse on or near the kidney 10.

With particular reference to Figure 2A, the renal artery 12, as with most arteries and arterioles, is lined with smooth muscle 34 that controls the diameter of the renal artery lumen 13. Smooth muscle, in general, is an involuntary non-striated muscle found within the media layer of large and small arteries and veins, as well as various organs. The glomeruli of the kidneys, for example, contain a smooth muscle-like cell called the mesangial cell. Smooth muscle is fundamentally different from skeletal muscle and cardiac muscle in terms of structure, function, excitation-contraction coupling, and mechanism of contraction.

Smooth muscle cells can be stimulated to contract or relax by the autonomic nervous system, but can also react on stimuli from neighboring cells and in response to hormones and blood borne electrolytes and agents (e.g., vasodilators or vasoconstrictors). Specialized smooth muscle cells within the afferent arteriole of the juxtaglomerular apparatus of kidney 10, for example, produces renin which activates the angiotension II system.

The renal nerves 14 innervate the smooth muscle 34 of the renal artery wall 15 and extend lengthwise in a generally axial or longitudinal manner along the renal artery wall 15. The smooth muscle 34 surrounds the renal artery circumferentially, and extends lengthwise in a direction generally transverse to the longitudinal orientation of the renal nerves 14, as is depicted in Figure 2B.

The smooth muscle 34 of the renal artery 12 is under involuntary control of the autonomic nervous system. An increase in sympathetic activity, for example, tends to contract the smooth muscle 34, which reduces the diameter of the renal artery lumen 13 and decreases blood perfusion. A decrease in sympathetic activity tends to cause the smooth muscle 34 to relax, resulting in vessel dilation and an increase in the renal artery lumen diameter and blood perfusion. Conversely, increased parasympathetic activity tends to relax the smooth muscle 34, while decreased parasympathetic activity tends to cause smooth muscle contraction.

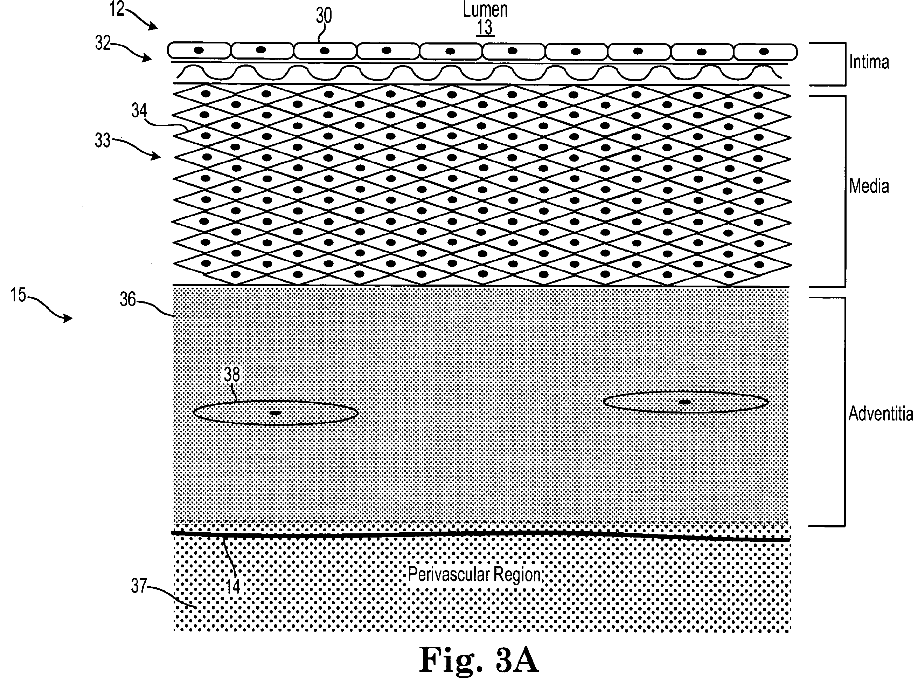

Figure 3A shows a segment of a longitudinal cross-section through a renal artery, and illustrates various tissue layers of the wall 15 of the renal artery 12. The innermost layer of the renal artery 12 is the endothelium 30, which is the innermost layer of the intima 32 and is supported by an internal elastic membrane. The endothelium 30 is a single layer of cells that contacts the blood flowing though the vessel lumen 13. Endothelium cells are typically polygonal, oval, or fusiform, and have very distinct round or oval nuclei. Cells of the endothelium 30 are involved in several vascular functions, including control of blood pressure by way of vasoconstriction and vasodilation, blood clotting, and acting as a barrier layer between contents within the lumen 13 and surrounding tissue, such as the membrane of the intima 32 separating the intima 32 from the media 34, and the adventitia 36. The membrane or maceration of the intima 32 is a fine, transparent, colorless structure which is highly elastic, and commonly has a longitudinal corrugated pattern.

Adjacent the intima 32 is the media 33, which is the middle layer of the renal artery 12. The media is made up of smooth muscle 34 and elastic tissue. The media 33 can be readily identified by its color and by the transverse arrangement of its fibers. More particularly, the media 33 consists principally of bundles of smooth muscle fibers 34 arranged in a thin plate-like manner or lamellae and disposed circularly around the arterial wall 15. The outermost layer of the renal artery wall 15 is the adventitia 36, which is made up of connective tissue. The adventitia 36 includes fibroblast cells 38 that play an important role in wound healing.

A perivascular region 37 is shown adjacent and peripheral to the adventitia 36 of the renal artery wall 15. A renal nerve 14 is shown proximate the adventitia 36 and passing through a portion of the perivascular region 37. The renal nerve 14 is shown extending substantially longitudinally along the outer wall 15 of the renal artery 12. The main trunk of the renal nerves 14 generally lies in or on the adventitia 36 of the renal artery 12, often passing through the perivascular region 37, with certain branches coursing into the media 33 to enervate the renal artery smooth muscle 34.

Embodiments of the disclosure may be implemented to provide varying degrees of denervation therapy to innervated renal vasculature. For example, embodiments of the disclosure may provide for control of the extent and relative permanency of renal nerve impulse transmission interruption achieved by denervation therapy delivered using a treatment apparatus of the disclosure. The extent and relative permanency of renal nerve injury may be tailored to achieve a desired reduction in sympathetic nerve activity (including a partial or complete block) and to achieve a desired degree of permanency (including temporary or irreversible injury).

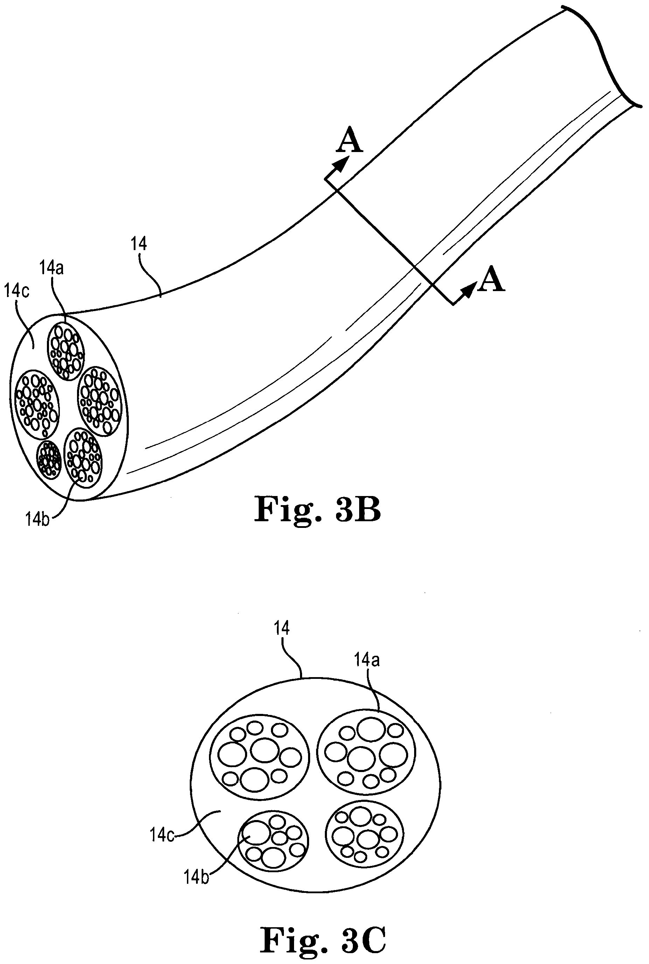

Returning to Figures 3B and 3C, the portion of the renal nerve 14 shown in Figures 3B and 3C includes bundles 14a of nerve fibers 14b each comprising axons or dendrites that originate or terminate on cell bodies or neurons located in ganglia or on the spinal cord, or in the brain. Supporting tissue structures 14c of the nerve 14 include the endoneurium (surrounding nerve axon fibers), perineurium (surrounds fiber groups to form a fascicle), and epineurium (binds fascicles into nerves), which serve to separate and support nerve fibers 14b and bundles 14a. In particular, the endoneurium, also referred to as the endoneurium tube or tubule, is a layer of delicate connective tissue that encloses the myelin sheath of a nerve fiber 14b within a fasciculus.

Major components of a neuron include the soma, which is the central part of the neuron that includes the nucleus, cellular extensions called dendrites, and axons, which are cable-like projections that carry nerve signals. The axon terminal contains synapses, which are specialized structures where neurotransmitter chemicals are released in order to communicate with target tissues. The axons of many neurons of the peripheral nervous system are sheathed in myelin, which is formed by a type of glial cell known as Schwann cells. The myelinating Schwann cells are wrapped around the axon, leaving the axolemma relatively uncovered at regularly spaced nodes, called nodes of Ranvier. Myelination of axons enables an especially rapid mode of electrical impulse propagation called saltation.

In some embodiments, a treatment apparatus of the disclosure may be implemented to deliver denervation therapy that causes transient and reversible injury to renal nerve fibers 14b. In other embodiments, a treatment apparatus of the disclosure may be implemented to deliver denervation therapy that causes more severe injury to renal nerve fibers 14b, which may be reversible if the therapy is terminated in a timely manner. In preferred embodiments, a treatment apparatus of the disclosure may be implemented to deliver denervation therapy that causes severe and irreversible injury to renal nerve fibers 14b, resulting in permanent cessation of renal sympathetic nerve activity. For example, a treatment apparatus may be implemented to deliver a denervation therapy that disrupts nerve fiber morphology to a degree sufficient to physically separate the endoneurium tube of the nerve fiber 14b, which can prevent regeneration and re-innervation processes.

By way of example, and in accordance with Seddon's classification as is known in the art, a treatment apparatus of the disclosure may be implemented to deliver a denervation therapy that interrupts conduction of nerve impulses along the renal nerve fibers 14b by imparting damage to the renal nerve fibers 14b consistent with neruapraxia. Neurapraxia describes nerve damage in which there is no disruption of the nerve fiber 14b or its sheath. In this case, there is an interruption in conduction of the nerve impulse down the nerve fiber, with recovery taking place within hours to months without true regeneration, as Wallerian degeneration does not occur. Wallerian degeneration refers to a process in which the part of the axon separated from the neuron's cell nucleus degenerates. This process is also known as anterograde degeneration. Neurapraxia is the mildest form of nerve injury that may be imparted to renal nerve fibers 14b by use of a treatment apparatus according to embodiments of the disclosure.

A treatment apparatus may be implemented to interrupt conduction of nerve impulses along the renal nerve fibers 14b by imparting damage to the renal nerve fibers consistent with axonotmesis. Axonotmesis involves loss of the relative continuity of the axon of a nerve fiber and its covering of myelin, but preservation of the connective tissue framework of the nerve fiber. In this case, the encapsulating support tissue 14c of the nerve fiber 14b is preserved. Because axonal continuity is lost, Wallerian degeneration occurs. Recovery from axonotmesis occurs only through regeneration of the axons, a process requiring time on the order of several weeks or months. Electrically, the nerve fiber 14b shows rapid and complete degeneration. Regeneration and re-innervation may occur as long as the endoneural tubes are intact.

A treatment apparatus may be implemented to interrupt conduction of nerve impulses along the renal nerve fibers 14b by imparting damage to the renal nerve fibers 14b consistent with neurotmesis. Neurotmesis, according to Seddon's classification, is the most serious nerve injury in the scheme. In this type of injury, both the nerve fiber 14b and the nerve sheath are disrupted. While partial recovery may occur, complete recovery is not possible. Neurotmesis involves loss of continuity of the axon and the encapsulating connective tissue 14c, resulting in a complete loss of autonomic function, in the case of renal nerve fibers 14b. If the nerve fiber 14b has been completely divided, axonal regeneration causes a neuroma to form in the proximal stump.

A more stratified classification of neurotmesis nerve damage may be found by reference to the Sunderland System as is known in the art. The Sunderland System defines five degrees of nerve damage, the first two of which correspond closely with neurapraxia and axonotmesis of Seddon's classification. The latter three Sunderland System classifications describe different levels of neurotmesis nerve damage.

The first and second degrees of nerve injury in the Sunderland system are analogous to Seddon's neurapraxia and axonotmesis, respectively. Third degree nerve injury, according to the Sunderland System, involves disruption of the endoneurium, with the epineurium and perineurium remaining intact. Recovery may range from poor to complete depending on the degree of intrafascicular fibrosis. A fourth degree nerve injury involves interruption of all neural and supporting elements, with the epineurium remaining intact. The nerve is usually enlarged. Fifth degree nerve injury involves complete transection of the nerve fiber 14b with loss of continuity.

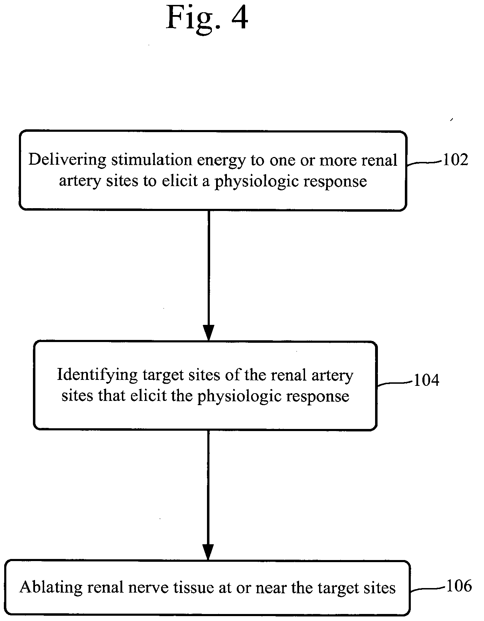

Turning now to Figure 4, there is illustrated various processes of a method for detecting and ablating renal nerves in accordance with embodiments of the disclosure. The method illustrated in Figure 4 involves delivering 102 stimulation energy to one or more renal artery sites to elicit a physiologic response from the patient but insufficient to ablate renal nerves. The method further involves identifying 104 target sites of the renal artery sites that elicit the physiologic response, and ablating 106 renal nerve tissue at or proximate the target sites, such as renal nerves included within the perivascular space adjacent the target sites. In some embodiments, the stimulation energy is delivered sequentially to the renal artery sites. In other embodiments, the stimulation energy is delivered concurrently to combinations of the renal artery sites.

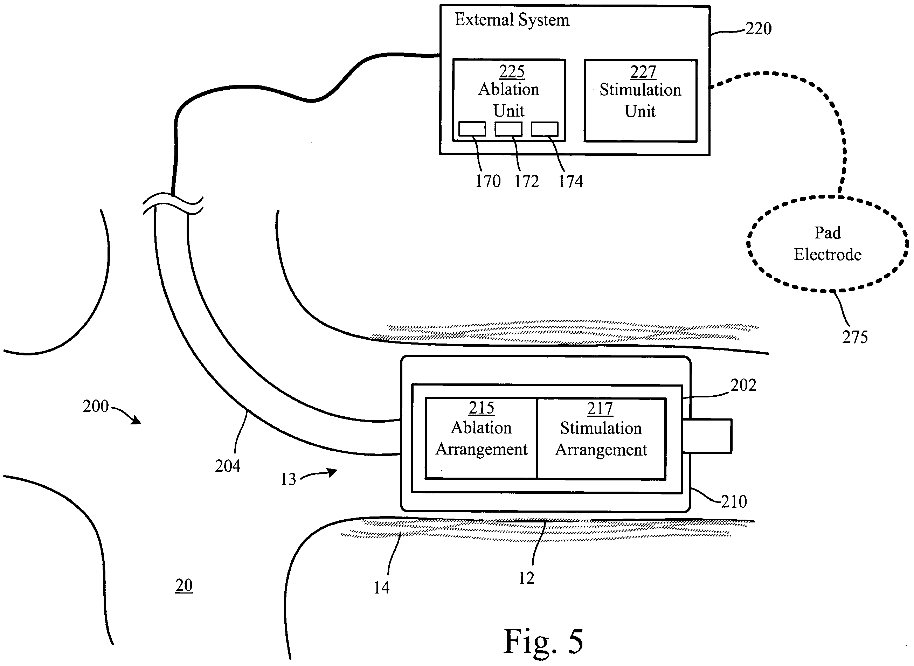

Figure 5 shows an apparatus for detecting and ablating renal nerves in accordance with various embodiments. In the embodiment shown in Figure 5, a catheter 200 includes a flexible shaft 204 having a proximal end, a distal end, and a length sufficient to access a patient's renal artery 12 relative to a percutaneous access location. The catheter 200 includes a treatment apparatus 202 provided at a distal end of the shaft 204. The treatment apparatus 202 is preferably centered within the renal artery 12 by an expandable support structure 210, which is transformable between a low-profile introduction configuration and a deployed configuration. In some embodiments, the support structure 210 includes an expandable wire or mesh structure, while in other embodiments, the support structure 210 includes a balloon structure.

The treatment apparatus 202 includes an ablation arrangement 215 and a stimulation arrangement 217, both of which are coupled to an external system 220. The external system 220 preferably includes a pad electrode 275 which can be placed on a portion of the patient's skin proximate the renal arteries. The pad electrode 275 serves as an external electrode for the various stimulation and ablation electrodes when operating in a unipolar mode. The stimulation arrangement 217 is coupled to the stimulation unit 227 of the external system 220. The stimulation unit 227 and the stimulation arrangement 217 cooperate to deliver a stimulation agent or agents selectively to each of a multiplicity of renal artery sites for purposes of eliciting a physiologic response from the patient. The stimulation arrangement 217 and stimulation unit 227 may be configured to deliver various types of stimulation agents, non-limiting examples of which include: electrical energy; optical energy; acoustic energy; mechanical force; vibration; thermal energy; a neurotransmitter; a chemical or pharmacological agent; pressure changes; osmotic changes; and pH changes. The stimulation arrangement 217 may be configured to deliver a stimulation agent or agents to a multiplicity of renal artery sites, either in a sequential manner or concurrently using a combination of renal artery sites.

During stimulation agent delivery, one or more *in vivo* and/or *ex vivo* sensors (along with clinician observation of the patient if desired), are monitored to determine which renal artery sites elicit a physiologic response. The renal artery sites that elicit a physiologic response are considered target sites for ablation. Non-limiting examples of physiologic response that can be sensed, detected or monitored include the following: pain; tingling; heat; pressure; changes in sympathetic signals or a resulting cascade of responses, including chemical or electrical changes; biometric indicators including skin conductivity or sweating; blood pressure, pulse or respiratory changes; changes in vascular or muscle tone; autonomic gastrointestinal activity; pupil response; and cardiac electrical activity.

After identifying which of the multiplicity of renal artery sites are considered target sites, the ablation unit 225 and the ablation arrangement 215 cooperate to ablate the target renal artery sites. The ablation unit 225 and the ablation arrangement 215 cooperate to deliver an ablative agent or agents to the target renal artery sites. Various types of ablative agents can be used to ablate the target renal artery sites, non-limiting examples of which include: electrical energy; optical energy for thermally ablating the renal nerves proximate the target sites; optical energy for forming micro-bubbles within renal nerve tissue proximate the target sites to mechanically disrupt nerve fibers and ganglia included within the renal nerve tissue upon implosion or explosion; acoustic energy for thermally ablating the renal nerves proximate the target sites; acoustic energy for forming micro-bubbles within renal nerve tissue proximate the target sites to mechanically disrupt nerve fibers and ganglia included within the renal nerve tissue upon implosion or explosion; mechanical loading or compressive force; cryothermal energy; thermal energy sufficient to cause coagulation, denaturation or necrosis; a neurotoxin or a venom; and an induced pH change sufficient to cause necrosis.

The ablation unit 225 shown in the embodiment of Figure 5 includes a control unit 170, energy delivery protocols 172, and a sensor unit 174. The control unit 170 is configured to control operation of the ablation unit 225, including implementation of one or more energy delivery protocols 172. The sensor unit 174 is coupled to one or more sensors which may be positioned at the treatment apparatus 202 or elsewhere on the catheter shaft 204, another catheter, or an external system, for sensing and monitoring one or more parameters during ablation. Useful sensors include temperature, impedance, voltage, acoustic, and tissue stiffness (elasticity) sensors, for example. In some embodiments, sensor data acquired by the sensor unit 174 during ablation allows the ablation unit 225 to control the ablation procedure in an automatic or semi-automatic mode.

The ablation arrangement 215 and the stimulation arrangement 217 are preferably centered within the lumen 13 of the renal artery 12 by the support structure 210. In some embodiments, the support structure 210 also serves to move one or both of the stimulation arrangement 217 and the ablation arrangement 215 into close proximity or contact with a lumen wall of the renal artery 12 when in the deployed configuration. The need for such radially outward movement of the stimulation arrangement 217 and/or the ablation arrangement 215 is dependent on the technology of each arrangement. In some embodiments, for example, a stimulation arrangement 217 and an ablation arrangement 215 that utilize radiofrequency electrodes can be positioned in close proximity or contact with the lumen wall of the renal artery 12 when the support structure 210 is in the deployed configuration. For some technologies, neither of the stimulation arrangement 217 and the ablation arrangement 215 needs to be moved radially outward by way of support structure deployment. In other embodiments, the ablation arrangement 215 remains relatively stationary at the shaft 204 of the ablation catheter 200, such as when the ablation arrangement 215 includes a high-intensity acoustic energy transducer (e.g., a high-intensity focused ultrasound (HIFU) device).

In accordance with other embodiments, the single catheter 200 shown in Figure 5 may instead the separated into two catheters, a simulation catheter and an ablation catheter. The stimulation catheter includes a flexible shaft 204 having a length sufficient to access a patient's renal artery relative to a percutaneous access location. A stimulation arrangement 217 is provided at the distal end of the shaft 204 and configured to deliver a stimulation agent to a multiplicity of renal artery sites. The ablation catheter also includes a flexible shaft, such as shaft 204, having a length sufficient to access the patient's renal artery relative to a percutaneous access location. An ablation arrangement 215 is provided at a distal end of the ablation catheter's shaft.

A first external system, stimulation unit 227, is configured to couple to the stimulation catheter and facilitate delivery of the stimulation agent selectively to a multiplicity of renal artery sites for eliciting of physiologic response from the patient. A second external system, ablation unit 225, is configured to couple to the ablation catheter and facilitate delivery of an ablative agent to target renal artery sites that elicited a physiologic response. In some embodiments, a combination unit is configured to incorporate both the functions of the stimulation unit 227 and the functions of the ablation unit 225.

According to some embodiments, the stimulation arrangement 217 is configured to deliver a first stimulation agent that causes pain and a second stimulation agent that blocks pain. The stimulation unit 227 cooperates with the stimulation arrangement 217 to selectively deliver the first and second stimulation agents to selectively cause pain and block pain for identifying renal artery sites that elicited physiologic response. According to other embodiments, the stimulation arrangement 217 is configured to deliver a first stimulation agent at a proximal renal artery location that causes temporary vasoconstriction by nerve activation of the arterial smooth muscle cells to facilitate locating of significant perivascular renal nerve bundles. The stimulation arrangement 217 is also configured to deliver a blocking agent at a multiplicity of distal renal artery locations to facilitate detection of efferent nerve locations.

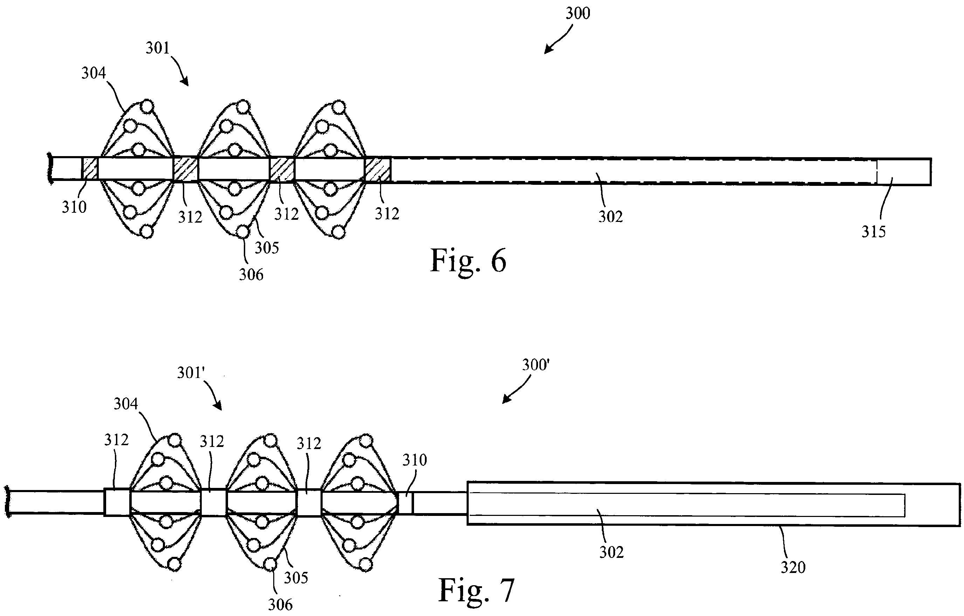

Referring now to Figure 6, there is illustrated an ablation catheter 300 which includes an electrode array structure 301 provided at a distal end of a flexible shaft 302 of the catheter 300. The flexible shaft 302 has a length sufficient to access a patient's renal artery relative to a percutaneous access location. The electrode array structure 302 shown in Figure 6 is dimensioned for deployment within the renal artery, and is transformable between a low-profile action configuration and a deployed configuration. The electrode array structure 301 includes at least one self-expanding basket structures 304 positioned in an axially spaced-apart relationship on the shaft 302. Three self-expanding basket structure 304 are illustrated in the embodiment of Figure 6, it being understood that fewer or more than three basket structures 304 may be provided at the distal end of a catheter shaft 302 (e.g., between two and five basket structures).

According to some embodiments, one end of the electrode array structure 301 is mounted on the shaft 302 at a fixed attachment member 310, while the other end of the electrode array structure 302 is coupled to a sliding attachment member 312 configured to translate longitudinally along the shaft 304 to facilitate radial expansion and reduction of the basket structures 304. In the embodiment shown in Figure 6, a distal end of the electrode array structure 301 is coupled to the fixed attachment member 310 and a proximal end of the electrode array structure 301 is coupled to the sliding attachment member 312. An additional sliding attachment member 312 is positioned between each pair of adjacent basket structures 304. It is understood that the locations of the fixed and sliding attachment arrangements 310 and 312 on the shaft 302 shown in Figure 6 may be reversed, such as in the embodiment shown in Figure 7 discussed hereinbelow.

A control element 315 of the ablation catheter 300 includes a distal end coupled to at least the most proximal sliding attachment member 312 of the electrode array structure 301. Moving the control element 315 in a proximal direction produces a tensile force on the electrode array structure 301, causing the basket structures 304 to assume a low-profile introduction configuration. Moving the control element 315 in the distal direction produces a compressive force on the electrode array structure 301, allowing the basket structures 304 to assume their deployed configuration, expanding radially toward the lumen wall of the renal artery.

The control element 315 is included in the embodiment shown in Figure 6 because the sliding attachment member 312 is positioned at the proximal end of the electrode array structure 301. In typical use, the electrode array structure 301 is positioned within a delivery sheath, such as the sheath 320 shown in Figure 7, with the basket structures 304 assuming their low-profile introduction configuration. After delivering the electrode array structure 301 into the renal artery, the sheath 320 is retracted to allow the basket structures 304 to assume their deployed configurations. After completion of renal nerve detection and ablation, the electrode array structure 301 is either drawn into the lumen of the sheath 320 or the sheath 320 is advanced over the electrode array structure 301 (or a combination of these movements).

Because the most proximal attachment member of the electrode array structure 301 is a sliding attachment member 312, attempting to slide the sheath 320 over the electrode baskets 304 would result in moving each of the sliding baskets 304 and associated sliding attachment members 312 in a distal direction towards the fixed attachment member 310 (due to the relative difference in the diameters of the baskets 304 and sheath 320). Accordingly, the control element 315 is used to facilitate retraction of the electrode array structure 301 into the sheath by application of a proximately directed tensile force.

As discussed previously, the basket structures 304 are preferably self-expanding structures. Suitable materials for constructing the self-expanding basket structures 304 include elastic or superelastic nitinol or a spring-like stainless steel. The self-expanding material of the basket structures 304 provides for easy self-deployment of the basket structures 304 when advanced out of the distal end of a delivery sheath 320, which is typically used to deliver the electrode array structure 301 to the renal artery. In some embodiments, use of a self-expanding basket structures 304 may obviate the need for a control element 315. Although the basket structures 304 are preferably self-expanding structures, the basket structures 304 may be formed from materials that do not have a self-expanding property, in which case the control element 315 is used for transforming the electrode array structure 301 between low-profile introduction and a deployed configurations. In such embodiments, each wire 305 of the basket structures 304 preferably has a pre-formed curve that causes the wire 3042 assume a preferred curved shape along a preferred bending plane when subject to radial expansion and reduction by use of the control element 315.

Each of the basket structures 304 includes a multiplicity of wires 305 having respective ends coupled to a fixed or slidable attachment member 310, 312. The wires 305 are formed to preferentially bend in a bending plane substantially normal to a longitudinal axis of the catheter shaft 302 in response to compressive and tensile forces. The cross-sectional shape of each wire 305, for example, can be round or other shape so as to impart a bias to help control the displacement and orientation of the wires 305 when actuated. The wires 305 can be formed with a pre-set shape using thermal or strain or other processing methods, to achieve the desired deployed configuration. The wires 305 are preferably formed from an electrically conductive metal or alloy. In some embodiments, portions of the wires 305 between an attachment member 310 or 312 and an electrode 306 can be covered with an insulating sleeve or coating. The wires 305 are arranged circumferentially around the shaft 302 of the catheter 300 in a spaced-apart relationship. Sufficient spacing is provided between adjacent wires 305 to ensure that the electrodes 306 do not contact one another during use. It is noted that a basket structure 304 with a braided or crossing wire structure can be used, for example, as long as the wires 305 are electrically insulated, and the bare electrodes 306 do not contact each other. This preferential bending aspect of the wires 305 provides for radial expansion and reduction of the basket structures 304 in response to compressive and tensile forces applied to the basket structures 304, respectively.

In the embodiment shown in Figure 6, each wire 305 supports one electrode 306. In embodiments where each electrode 306 can be activated and deactivated independent of other electrodes 306, each wire 305 (or a conductor coupled to each wire 305) extends along the length of the shaft 302 to the proximal end of the catheter 300. The wires 305 or conductors extending along the length of the shaft 302 are insulated from one another, preferably by use of an insulating sleeve or coating on each wire 305 or conductor. The electrodes of each basket structure 304 may be offset axially and/or circumferentially from one another. This offset electrode arrangement of each basket structure 304 provides for delivery of stimulation energy to a multiplicity of axially and/or circumferentially spaced-apart discrete locations of the renal artery wall. An offset electrode arrangement can also be useful in aiding the packing of the structure to achieve a lower profile for introduction.

In Figure 7, there is shown an ablation catheter 300' which is similar to that illustrated in Figure 6. According to the embodiment shown in Figure 7, an electrode array structure 301 includes three self-expanding basket structures 304. The electrode array structure 301 has a distal end coupled to a sliding attachment member 312 and a proximal end coupled to a fixed attachment member 310. This arrangement of sliding and fixed attachment members 312, 310 is a reverse of that shown in Figure 6. Because the most proximal attachment member is a fixed attachment member 310, the control element 315 shown in Figure 6 is not needed in the embodiment illustrated in Figure 7.

It is noted that in some embodiments, a control element 315 can be incorporated into the embodiment shown in Figure 7, with the distal end of the control element 315 coupled to the most distal sliding attachment member 312. The control element 315, according to such embodiments, may be configured as a relatively small wire that extends through a lumen of the shaft 320 and extends through a longitudinal slot situated adjacent the most distal sliding attachment member 312. The extent of the longitudinal slot dictates the extent of longitudinal travel of the sliding attachment number 312.

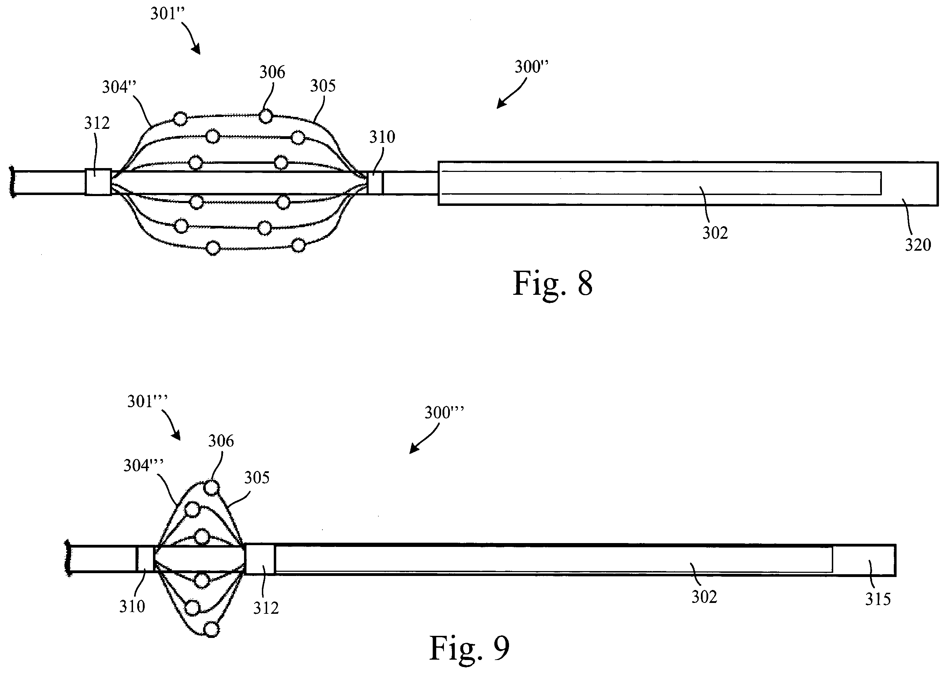

Figure 8 illustrates an ablation catheter 300" configured for renal nerve detection and ablation in accordance with various embodiments. In Figure 8, the electrode array structure 301" includes a single self-expanding basket structure 304" having long longitudinal aspect relative to its radial aspect. For example, the longitudinal aspect of the basket structure 304"when in a deployed configuration can be 2 to 4 times greater than its radial aspect. Each of the wires 305 of the basket structure 304" includes a multiplicity of electrodes 306, such as two electrodes 306. The multiple electrodes 306 supported by each wire 305 are connected in series. However, each electrode pair provided on each wire 305 is preferably energizable independent of other electrode pairs of other wires 305. Alternatively, additional insulation and conductor elements can be provided along each wire 305 to energize each electrode 306 independently.

In the embodiment shown in Figure 8, the proximal end of the electrode array structure 301" is coupled to a fixed attachment member 310, and the distal end of the electrode array structure 301" is coupled to a sliding attachment number 312. A delivery sheath 320 is used to constrain the electrodes 306 and basket structure 304" in a low-profile introduction configuration during delivery and retraction to and from the renal artery. Because the proximal attachment member 310 is fixed to the shaft 302, a control member 315 is not required, but may be included and attached to the distal sliding attachment member 312 if desired. It is understood that the proximal and distal arrangements of 310 and 312 may be reversed, which would require a control element 315 for actuation.

Figure 9 illustrates an ablation catheter 300'" for detecting and ablating renal nerves in accordance with various embodiments. In Figure 9, the electrode array structure 301"' includes a single, relatively short self-expanding basket structure 304"'. The proximal end of the electrode array structure 301'" is coupled to a sliding attachment number 312, while the distal end of the electrode array structure 301'" is coupled to a fixed attachment member 310. A control element 315 has a distal end coupled to the sliding attachment members 312. In the embodiment shown in Figure 9, the basket structure 304'" has the advantage of a simpler design and shorter basket structure 304"', but would require accurate device repositioning to multiple axial locations of the renal artery if staggered axial positioning is desired. It is noted that the embodiment shown in Figure 9 and in other figures could have axially aligned electrodes for creating a circumferential lesion or lesions.

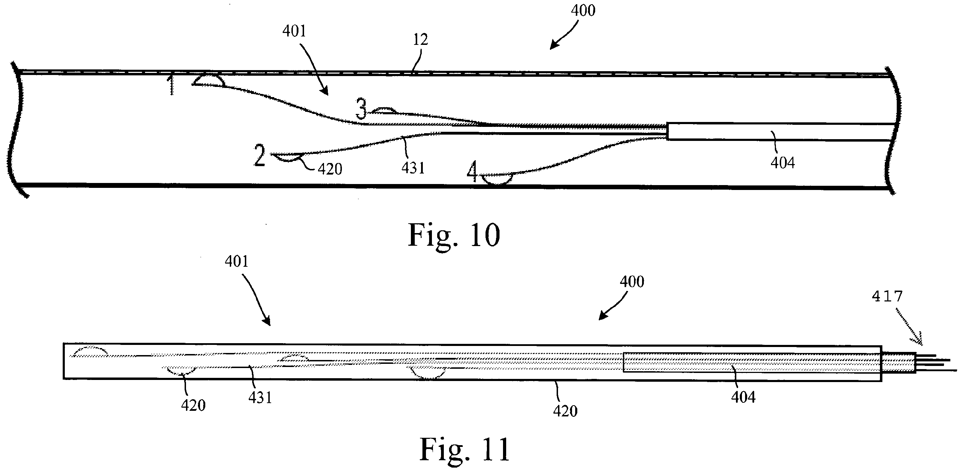

Referring now to Figure 10, there is illustrated a renal nerve detection and ablation catheter 400 in accordance with various embodiments of the disclosure. Figure 11 shows the catheter 400 of Figure 10 in a low-profile introduction configuration due to being constrained in a lumen of a delivery sheath 420. The ablation catheter 400 shown in Figure 10 includes a shaft 404 having a lumen dimensioned to receive an electrode array structure 401. The electrode array structure 401 includes a multiplicity of resilient support members 431 and an electrode assembly 420 supported by a respective support member 431. Each of the support members 431 defines an apposition member or wire that preferably incorporates a pre-formed curve. In some embodiments, the electrode array structure 401 is fixedly disposed within the lumen of the shaft 404 and is delivered to the renal artery 12 using the flexible sheath 420. In other embodiments, the electrode array structure 401 is displaceably disposed within the lumen of the shaft 404, and can be retracted within and extended beyond the distal end of the catheter shaft 404. In further embodiments, individual or pairs of the support members 431 is/are displaceable within a respective lumen of the shaft 404. The ablation catheter 400 can be configured as a guiding catheter or may be delivered to the renal artery 12 using a guiding catheter and/or a flexible delivery sheath 420. The resilient support members 431 can be constrained to a low profile when encompassed by a wall of a removable sheath 420 or a lumen wall of the shaft 404 and, when removed from the removable sheath 420 or lumen of the shaft 404, the resilient support members 431 expand outwardly and assume a shape of the pre-formed curve.

In Figure 10, four electrodes 420 are shown for purposes of explanation. It is understood that fewer or greater than four electrodes 420 may be provided in various embodiments, and each electrode 420 can be configured for use at a discrete axial and circumferential location of the renal artery 12. For example, up to six or eight electrodes 420 and corresponding support members 431 may be provided. Each electrode 420/resilient support member 431 is coupled to a conductor 417 which extends along the length of the shaft 404 to the proximal end of the catheter 400. Each of the conductors 417 includes an insulating sleeve or coating.

The resilient support members 431 are constructed to be collapsible when encompassed by a wall of a removable sheath 420 or lumen wall of the shaft 404, and expand outwardly when removed from the removable sheath 420 or extended from the shaft lumen. The resilient support members 431 can be constructed as a single or a multiple element structure, providing high or superelastic properties and good electrical conduction properties. For example, the resilient support members 431 can be constructed to have a shape memory, such that the resilient support members 431 expand outwardly and assume a shape of the pre-formed curve when in a deployed configuration. The resilient support members 431 may be constructed as apposition wires fabricated from superelastic nickel titanium alloy, stainless steel, or other spring-like or self-expanding type materials, and are formed so that they are biased outward to force the electrodes 420 against the wall of the renal artery 12. The resilient support members 431 can include both a structural spring-like element (such as elastic or superelastic nitinol or a spring-like stainless steel) and a superior electrical conductor (such as copper or platinum), or a single element can provide both the spring-like support and the electrical conductivity properties.

According to various embodiments, each of the resilient support members 431 is constructed from an electrically conductive material and configured as a wire. Each of the conductive resilient support members 431 is coupled to an electrical conductor disposed in a lumen of the catheter shaft 404. The conductive resilient support members 431 preferably include an electrically insulating material or coating, and the electrical conductors coupled to the resilient support members 431 are electrically insulated from one another (e.g., by way of insulating material/coating or separate lumens within the shaft 404). Electrically insulating the respective resilient support members 431 provides for individual activation and deactivation of each electrode 420 in accordance with a predefined energy delivery protocol.

Embodiments of the disclosure can include one or more systems, devices, sensors, features and/or functions disclosed in the following commonly-owned co-pending U.S. Patent Publication Nos.; 20110257523; 20110257641; 20110263921; 20110264086; and 20110264116; and U.S. patent Application Serial Nos. 13/157,844 filed June 10, 2011; 13/188,677 filed July 22, 2011; 13/193,338 filed July 28, 2011; 13/184,677 filed July 18, 2011; 13/228,233 filed September 8, 2011; 13/281,962 filed October 26, 2011; 13/243,114 filed September 23, 2011; 13/243,724 filed September 23, 2011; 13/295,185 filed November 14, 2011; and 13/243,729 filed September 23, 2011.

It is to be understood that even though numerous characteristics of various embodiments have been set forth in the foregoing description, together with details of the structure and function of various embodiments, this detailed description is illustrative only, and changes may be made in detail, especially in matters of structure and arrangements of parts illustrated by the various embodiments to the full extent indicated by the broad general meaning of the terms in which the appended claims are expressed.

## Claims

1. An apparatus, comprising:
a catheter (300) comprising a flexible shaft (302) having a proximal end, a distal end, and a length sufficient to access a patient's renal artery relative to a percutaneous access location;
an electrode array (301) provided at the distal end of the shaft and dimensioned for deployment within the renal artery, the electrode array comprising a plurality of self-expanding basket structures (304) supporting a plurality of spaced-apart electrodes (306) positionable at a plurality of renal artery sites at or near a wall of the renal artery; and
an external system (220) configured to couple to the catheter and deliver stimulation energy selectively to the plurality of electrodes for eliciting a physiologic response from the patient but insufficient to ablate renal nerves, the external system further configured to deliver high-frequency electrical energy to electrodes at target renal artery sites that elicit the physiologic response for ablating renal nerves proximate the target sites;
wherein one of a distal end and a proximal end of the basket structures is fixedly mounted (310) on the shaft (302) and a sliding attachment member (312) is situated between each of the basket structures (304) along the shaft and at the other of the distal and proximal ends of the basket structures said sliding attachment members (312) configured to translate longitudinally along the shaft (302) to facilitate radial expansion and reduction of the basket structure;
a control element having a distal end coupled to the sliding attachment member and an actuatable proximal end.

2. The apparatus of claim 1, wherein the electrodes of the electrode array are respectively activatable and deactivatable individually or in selected combinations.

3. The apparatus of claim 1, further comprising
a control element has an actuatable proximal end and a distal end coupled to at least one of the sliding attachment members wherein the sliding attachment members are configured to translate longitudinally along the shaft to facilitate radial expansion and reduction of the basket structures.

4. The apparatus of claim 1, comprising an external sheath having a lumen dimensioned to receive the electrode array.

5. The apparatus of claim 1, wherein the stimulation energy is delivered sequentially to the renal artery sites or concurrently to combinations of the renal artery sites.

6. The apparatus of claim 1, wherein the stimulation energy has one or more of a frequency, amplitude, and pattern that enhances eliciting of the physiologic response.

7. The apparatus of claim 1, wherein the energy delivery system is configured to selectively deliver stimulation energy to the renal artery following renal nerve ablation to determine effectiveness of the ablation.

8. The apparatus of claim 1, comprising one or more implantable or external sensor configured to sense for eliciting of the physiologic response.

9. The apparatus of claim 1, wherein the physiologic response comprises one or more of pain, tingling, heat, and pressure.

10. The apparatus of claim 1, wherein the physiologic response comprises changes in sympathetic signals or a resulting cascade of responses, including chemical or electrical changes, biometric indicators including skin conductivity or sweating, blood pressure, pulse or respiratory changes, changes in vascular or muscle tone, autonomic gastrointestinal activity, pupil response, and cardiac electrical activity.

11. The apparatus of claim 1, wherein ablating renal nerve tissue at the target sites is performed after completion of target site identification without repositioning the electrode array.

12. The apparatus of claim 1, wherein:
the electrode array is transformable between a low-profile introduction configuration and a deployed configuration; and
the spaced-apart electrodes are positionable at the plurality of renal artery sites when the electrode array is in the deployed configuration.

## Patentansprüche

1. Vorrichtung, die aufweist:
einen Katheter (300), der einen flexiblen Schaft (302) mit einem proximalen Ende, einem distalen Ende und einer Länge aufweist, die ausreicht, relativ zu einer perkutanen Zugangsstelle eine Nierenarterie eines Patienten anzusteuern;
eine Elektrodenanordnung (301), die am distalen Ende des Schafts vorgesehen und zum Einsatz in der Nierenarterie ausgelegt ist, wobei die Elektrodenanordnung mehrere von selbstexpandierende Korbstrukturen (304) aufweist, die mehrere beabstandete Elektroden (306) halten, die an mehreren Nierenarterienstellen an oder nahe einer Wand der Nierenarterie positionierbar sind; und
ein externes System (220), das konfiguriert ist, mit dem Katheter zu koppeln und zum Hervorrufen einer physiologischen Reaktion vom Patienten selektiv Stimulationsenergie an die mehreren Elektroden abzugeben, die jedoch nicht ausreicht, um Nierennerven abzutragen, wobei das externe System ferner konfiguriert ist, elektrische Hochfrequenzenergie an Elektroden an Zielnierenarterienstellen, die die physiologische Reaktion hervorrufen, zum Abtragen von Nierennerven benachbart zu den Zielstellen abzugeben;
wobei eines von einem distalen Ende und einem proximalen Ende der Korbstrukturen fest am Schaft (302) angebracht ist (310) und ein gleitendes Befestigungselement (312) zwischen jeder der Korbstrukturen (304) längs des Schafts und an dem anderen des distalen und proximalen Endes der Korbstrukturen angeordnet ist, wobei die gleitenden Befestigungselemente (312) konfiguriert sind, sich longitudinal längs des Schafts (302) zu verschieben, um die radiale Ausdehnung und Verkleinerung der Korbstruktur zu erleichtern;
ein Steuerelement, das ein distales Ende, das mit dem gleitenden Befestigungselement gekoppelt ist, und betätigbares proximales Ende aufweist.

2. Vorrichtung nach Anspruch 1, wobei die Elektroden der Elektrodenanordnung jeweils einzeln oder in ausgewählten Kombinationen aktivierbar und deaktivierbar sind.

3. Vorrichtung nach Anspruch 1, die ferner aufweist
ein Steuerelement, das ein betätigbares proximales Ende und ein distales Ende aufweist, das an mindestens eines der gleitenden Befestigungselemente gekoppelt ist, wobei die gleitenden Befestigungselemente konfiguriert sind, sich longitudinal längs des Schafts zu verschieben, um die radiale Ausdehnung und Verkleinerung der Korbstrukturen zu erleichtern.

4. Vorrichtung nach Anspruch 1, die eine externe Hülle mit einem Lumen aufweist, das ausgelegt ist, die Elektrodenanordnung aufzunehmen.

5. Vorrichtung nach Anspruch 1, wobei die Stimulationsenergie sequentiell an die Nierenarterienstellen oder gleichzeitig an Kombinationen der Nierenarterienstellen abgegeben wird.

6. Vorrichtung nach Anspruch 1, wobei die Stimulationsenergie eine Frequenz und/oder eine Amplitude und/oder ein Muster aufweist, das das Hervorrufen der physiologischen Reaktion verstärkt.

7. Vorrichtung nach Anspruch 1, wobei das Energieabgabesystem konfiguriert ist, anschließend an die Nervenabtragung selektiv Stimulationsenergie an die Nierenarterie abzugeben, um die Wirksamkeit der Abtragung festzustellen.

8. Vorrichtung nach Anspruch 1, die einen oder mehrere implantierbare oder externe Sensoren aufweist, die konfiguriert sind, das Hervorrufen der physiologischen Reaktion abzutasten.

9. Vorrichtung nach Anspruch 1, wobei die physiologische Reaktion Schmerzen und/oder Prickeln und/oder Wärme und/oder Druck aufweist.

10. Vorrichtung nach Anspruch 1, wobei die physiologische Reaktion Änderungen der sympathischen Signale oder eine resultierende Kaskade von Reaktionen, die chemische oder elektrische Änderungen umfassen, biometrische Indikatoren, die die Hautleitfähigkeit oder Schwitzen umfassen, den Blutdruck, Puls- oder Atmungsänderungen, Änderungen des vaskulären oder Muskeltonus, der autonomen Magen-Darm-Aktivität, der Pupillenreaktion und der elektrischen Herzaktivität aufweist.

11. Vorrichtung nach Anspruch 1, wobei das Abtragen des Nierennervengewebes an den Zielstellen nach der Beendigung der Zielstellenerkennung ohne Neuanordnung der Elektrodenanordnung durchgeführt wird.

12. Vorrichtung nach Anspruch 1, wobei:
die Elektrodenanordnung zwischen einer Niederprofil-Einführungskonfiguration und einer entfalteten Konfiguration umwandelbar ist; und
die beabstandeten Elektroden an mehreren Nierenarterienstellen positionierbar sind, wenn sich die Elektrodenanordnung in der entfalteten Konfiguration befindet.

## Revendications

1. Dispositif, comprenant :
un cathéter (300) comportant une tige flexible (302) ayant une extrémité proximale, une extrémité distale, et une longueur suffisante pour accéder à une artère rénale d'un patient depuis un site d'accès percutané ;
un réseau d'électrodes (301) prévu à l'extrémité distale de la tige et dimensionné pour un déploiement à l'intérieur de l'artère rénale, ledit réseau d'électrodes comprenant une pluralité de structures en corbeille auto-expansibles (304) supportant une pluralité d'électrodes (306) espacées, positionnables sur une pluralité de sites artériels rénaux sur une paroi d'artère rénale ou à proximité de celle-ci ; et
un système externe (220) prévu pour être relié au cathéter et délivrer sélectivement un courant de stimulation à la pluralité d'électrodes, permettant de provoquer une réaction physiologique du patient mais insuffisant pour l'ablation de nerfs rénaux, ledit système externe étant prévu en outre pour délivrer un courant électrique à haute fréquence aux électrodes aux sites artériels rénaux cibles, laquelles provoquent la réaction physiologique pour l'ablation de nerfs rénaux à proximité des sites cibles ;
où une extrémité distale ou une extrémité proximale des structures en corbeille est fixement montée (310) sur la tige (302), et des éléments de fixation coulissant (312) sont situés chacun entre deux structures en corbeille (304) le long de la tige et sur l'autre extrémité, distale ou proximale des structures en corbeille, lesdits éléments de fixation coulissants (312) étant prévus pour être mobiles longitudinalement sur la tige (302) afin de faciliter l'expansion radiale et la réduction de la structure en corbeille ;
un élément de commande ayant une extrémité distale reliée à l'élément de fixation coulissant et une extrémité proximale actionnable.

2. Dispositif selon la revendication 1, où les électrodes du réseau d'électrodes sont respectivement activables et désactivables, individuellement ou par combinaisons sélectionnées.

3. Dispositif selon la revendication 1, comprenant en outre
un élément de commande ayant une extrémité proximale actionnable et une extrémité distale reliée à au moins un des éléments de fixation coulissants, les éléments de fixation coulissants étant prévus pour être mobiles longitudinalement sur la tige afin de faciliter l'expansion radiale et la réduction des structures en corbeille.

4. Dispositif selon la revendication 1, comprenant une gaine externe ayant une lumière dimensionnée pour recevoir le réseau d'électrodes.

5. Dispositif selon la revendication 1, où le courant de stimulation est délivré séquentiellement aux sites artériels rénaux ou concurremment à des combinaisons des sites artériels rénaux.

6. Dispositif selon la revendication 1, où le courant de stimulation a une fréquence et/ou une amplitude et/ou une courbe qui facilite le déclenchement de la réaction physiologique.

7. Dispositif selon la revendication 1, où le système de délivrance de courant est prévu pour délivrer sélectivement un courant de stimulation à l'artère rénale consécutivement à l'ablation d'un nerf rénal afin de déterminer l'efficacité de l'ablation.

8. Dispositif selon la revendication 1, comprenant un ou plusieurs capteurs implantables ou externes prévus pour détecter le déclenchement de la réaction physiologique.

9. Dispositif selon la revendication 1, où la réaction physiologique se traduit par une douleur et/ou des picotements et/ou une sensation de chaleur et/ou de pression.

10. Dispositif selon la revendication 1, où la réaction physiologique se traduit par une variation des signaux sympathiques ou une cascade résultante de réactions, comprenant des modifications chimiques ou électriques, des indicateurs biométriques incluant la conductivité cutanée ou la transpiration, la pression sanguine, des variations de pouls ou de rythme respiratoire, des variations du tonus vasculaire ou musculaire, de l'activité gastro-intestinale autonome, des réflexes pupillaires, et de l'activité électrique cardiaque.

11. Dispositif selon la revendication 1, où l'ablation du tissu nerveux rénal sur les sites cibles est exécutée après identification de site cible sans repositionnement du réseau d'électrodes.

12. Dispositif selon la revendication 1, où :
le réseau d'électrodes est commutable entre une configuration d'introduction à profil contracté et une configuration déployée ; et où
les électrodes espacées sont positionnables sur la pluralité de sites artériels rénaux quand le réseau d'électrodes est en configuration déployée.
